# EUROPEAN PATENT SPECIFICATION

(11) **EP 3 980 091 B1**
(45) Date of publication and mention of the grant of the patent: **28.02.2024**
(21) Application number: 20730334.8
(22) Date of filing: 03.06.2020
(51) Int. Cl.: A61M 1/16

(54) **CARTRIDGE FOR THE PREPARATION OF A DIALYSIS SOLUTION**
KARTUSCHE ZUR HERSTELLUNG EINER DIALYSELÖSUNG
CARTOUCHE POUR LA PRÉPARATION D'UNE SOLUTION DE DIALYSE

(30) Priority: 07.06.2019 IT 201900008403
(43) Date of publication of application: 13.04.2022
(73) Proprietor: Allmed Medical Care Holdings Limited, London W4 5YS (GB)
(72) Inventor: PÖTZSCH, Thomas, London W4 5YS (GB); BLUDSZUWEIT-PHILIPP, Catrin, London W4 5YS (GB)
(74) Representative: Brunacci, Marco
(86) International application number: PCT/IB2020/055223
(87) International publication number: WO 2020/245734

(56) References cited:
- WO-A1-00/57935
- WO-A1-2006/120415
- WO-A1-2012/041790
- DE-U1- 29 718 407
- DE-U1-202014 104 252

## Description

### Technical Field

The present invention refers to a cartridge to contain substances for the preparation of a dialysis solution.

### Background Art

Generally, a dialysis solution is a solution of electrolytes used in the treatment of a patient's blood which need to be purified by means of an extracorporeal process.

Dialysis solutions consist of buffered solutions of an appropriate buffering agent, such as sodium bicarbonate, sodium acetate, etc., in a solvent, such as purified water.

In order to reduce the risks due to microbial contamination of aqueous solutions, it is increasingly common to use cartridges containing the buffering agent in solid form, which is subsequently dissolved during the dialysis treatment at the moment of use.

Cartridges for the preparation of dialysis solutions are installed in medical equipment in which the solvent is introduced inside the cartridge for the extemporaneous preparation of the buffered solution.

Cartridges of the known type are generally made of plastic material and consist of a hollow body, defining a cavity inside which the buffering agent is contained, provided with a solvent inlet mouth and a dialysis solution outlet mouth.

The hollow body of the known cartridge has a truncated-cone shape and its upper portion, arranged in proximity of the inlet mouth is the larger one and, therefore, also the weaker one.

Such zone is more susceptible to external stresses due to accidental impacts occurring, for example, during transport, storage and/or to pressure forces applied during installation on the medical equipment.

A typology of cartridges of the known type provides that the reinforced portion has a thicker wall than the other portions, in order to better withstand the stresses.

However, this solution involves an increase in manufacturing costs which leads to a higher offer price, making the products less interesting for the market.

A further typology of cartridges, on the other hand, requires that the reinforced portion presents some ribs, on its internal surface, in order to increase the mechanical resistance of this portion.

However, these ribs cause a local concentration of mechanical stresses which can lead to cracks and damages to the cartridge itself.

The prior art document WO 2012/041790 discloses a cartridge for the preparation of a dialysis solution comprising portions which are reinforced by means of stiffening ribs.

The document DE 29718407 discloses a cartridge for the preparation of a dialysis solution comprising an hollow body and a lid, associated to the hollow body and defining an inlet mouth for the inflow of a solvent into the hollow body, where the outer surface of such a lid has a waved portion so as to facilitate its gripping by a user.

### Disclosure of the Invention

The main purpose of the present invention is to provide a cartridge for the preparation of a dialysis solution in which the risk of breakage and damage due to external stresses is highly reduced with respect of the known type.

Within this purpose, one object of the present invention is to provide a cartridge for the preparation of a dialysis solution in which the reinforced portion avoids the local concentration of mechanical stresses.

One object of the present invention is to provide a cartridge for the preparation of a dialysis solution having manufacturing costs lower than those of the cartridges of the known type.

Another object of the present invention is to provide a cartridge for the preparation of a dialysis solution which allows to overcome the mentioned drawbacks of the prior art within the ambit of a simple, rational, easy and effective use as well as low cost solution.

The aforementioned objects are achieved by the present cartridge for the preparation of a dialysis solution according to claim 1.

### Brief Description of the Drawings

Other characteristics and advantages of the present invention will become better evident from the description of a preferred but not exclusive embodiment of a cartridge for the preparation of a dialysis solution, illustrated by way of an indicative, but not limitative example in the accompanying drawings in which:

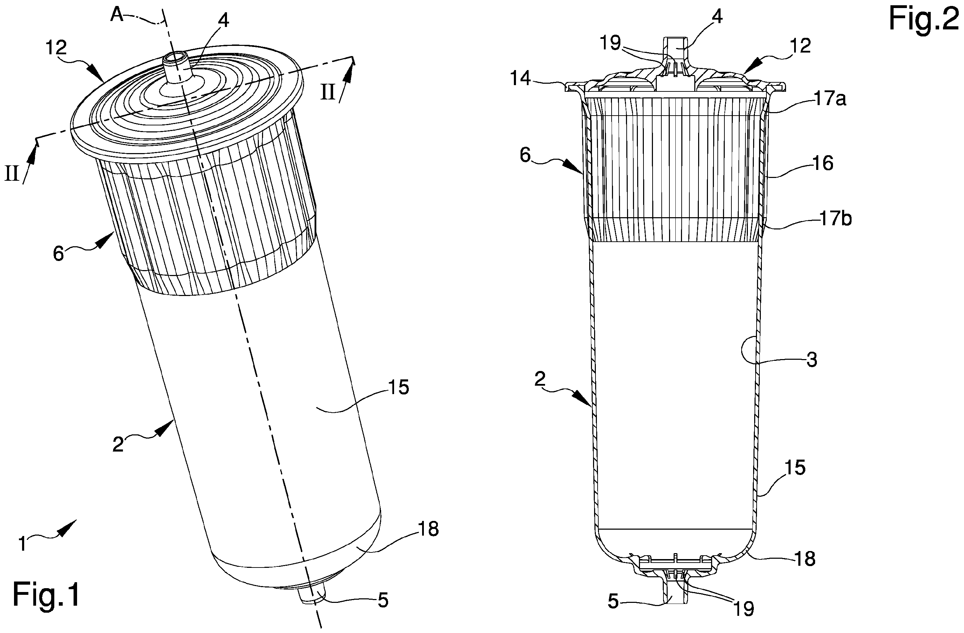

Figure 1 is an axonometric view of the cartridge according to the invention;
Figure 2 is a longitudinal sectional view of the cartridge of Figure 1 on the plane II-II;

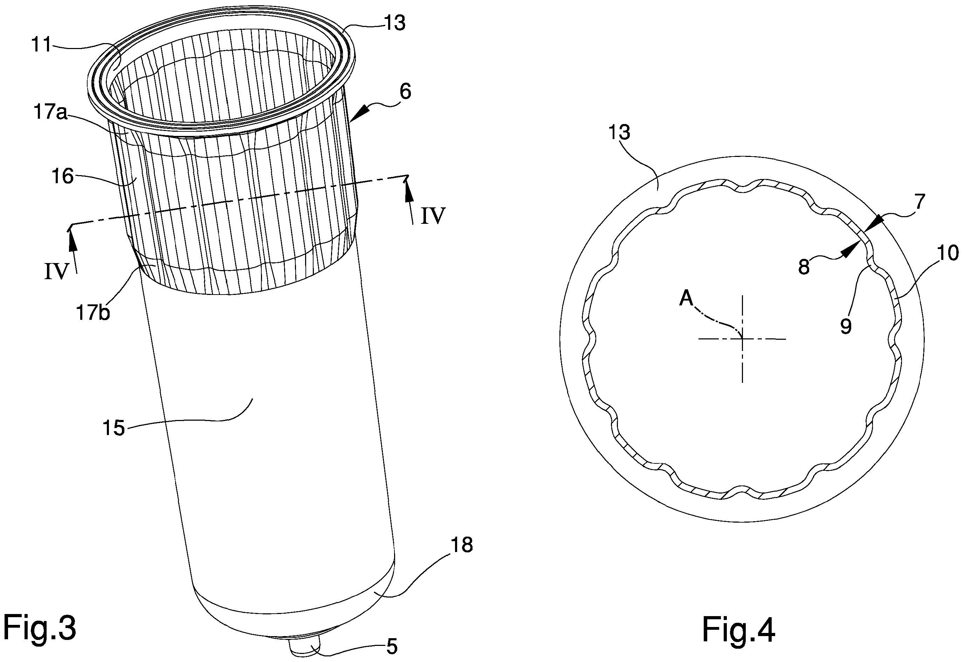

Figure 3 is an axonometric view of a component of the cartridge;
Figure 4 is a transversal sectional view of the component of Figure 3 on the plane IV-IV.

### Ways of carrying out the Invention

With particular reference to these figures, a cartridge for the preparation of a dialysis solution is globally indicated by reference number 1.

The cartridge 1 according to the present invention is intended to be used in a medical equipment for the extracorporeal treatment of a patient's blood, in particular a haemodialysis equipment.

The cartridge 1 is conveniently realized in plastic material, such as polyethylene, polypropylene, or any material suitable for the purpose of the present invention.

The cartridge 1 for the preparation of a dialysis solution comprises at least a hollow body 2, defining at least a cavity 3 adapted to contain a buffering agent. In particular, the buffering agent is in solid form and is chosen between sodium acetate, sodium bicarbonate or the like.

The use of the buffering agent in solid form significantly reduces the risk of bacterial growth with respect to a ready-to-use solution or a concentrated solution.

Preferably, the cartridge 1 has a capacity of between 600 to 1350 grams depending on the type of treatment to be performed.

The hollow body 2 comprises at least an inlet mouth 4, for the inflow of a solvent in said cavity 3, and an outlet mouth 5 for the outflow of said dialysis solution from said cavity 3.

Preferably, the solvent is water for injection.

More in detail, the solvent and the buffering agent, when in mutual contact, form the aforementioned dialysis solution which is then introduced in the medical equipment.

Advantageously, the hollow body 2 has elongated shape and the mouths 4,5 are placed at opposite ends of the hollow body itself, along the longitudinal axis A.

Conveniently, the mouths 4,5 are equipped with filters, for example of the type of 0,06 mm filters, to prevent particulate material, which is, for example, undissolved buffering agent particles, from flowing out of the cartridge 1.

The hollow body 2 also comprises at least a reinforced portion 6 interposed between said inlet mouth 4 and said outlet mouth 5.

Usefully, the reinforced portion 6 is disposed in proximity of the inlet mouth 4.

The reinforced portion 6 is adapted to ensure high mechanical strength to the cartridge itself and, thus, the risk of breakage and damage due to external stresses is highly reduced.

According to the invention, the wall of the reinforced portion 6 is wave-shaped. In particular, the reinforced portion 6 comprises an outer surface 7 and an inner surface 8 being both wave-shaped.

More in detail, the outer surface 7 and the inner surface 8 are substantially smooth and provided with a series of indentations 9 and protrusions 10 arranged alternately and in succession the one another.

Advantageously, the thickness of the wave-shaped wall of the reinforced portion 6 is substantially constant so as to avoid the local concentration of mechanical stresses and to increase its mechanical resistance.

Furthermore, the particular conformation of the reinforced portion 6 ensures the desired strength to the cartridge 1 with the minimum possible wall thickness, so as to reduce the related manufacturing costs.

Furthermore, the wave-shaped wall of the reinforced portion 6 increases the manual hold of the cartridge 1 and helps to improve its grip, especially when handled with protective gloves.

Usefully, the wave-shaped wall develops transversally with respect to the longitudinal axis A of the hollow body 2.

In particular, the reinforced portion 6 has a substantially circular transversal section and the wave-shaped wall develops along the circumference of said circular transversal section.

The reinforced portion 6 defines an opening access 11 for introducing the buffering agent into the cavity 3.

Advantageously, the hollow body 2 comprises a lid 12 associated to the reinforced portion 6 for closing the opening access 11 where the lid 12 defines said inlet mouth 4.

The lid 12 is separated from the reinforced portion 6. In the preferred for of embodiment disclosed in the figures, only the outer surface 7 and the inner surface 8 are wave-shaped, while those of the lid 12 are of the smooth type. However, alternative form of embodiments in which also the outer surface and the inner surface of the lid 12 are wave-shaped, are not excluded.

The hollow body 2 also comprises a circular flange 13, associated to the reinforced portion 6, in proximity of the opening access 11, and adapted to be welded to the lid 12 defining an air-tight welded zone 14.

Usefully, the hollow body 2 comprises a main portion 15 associated to the reinforced portion 6.

The thickness of the wall of the main portion 15 substantially corresponds to the thickness of the wall of the reinforced portion 6.

The reinforced portion 6 comprises a main sector 16 and at least a junction sector 17a,17b connecting the main sector 16 to at least one between the opening access 11 and the main portion 15.

The junction sector 17a,17b is truncated-cone shaped converging towards the main portion 15 and is inclined with respect to the main sector 16.

In other words, the junction sector 17a,17b has a different inclination with respect to the main sector 16.

Advantageously, the reinforced portion 6 comprises two of said junction sectors 17a,17b, of which an upper junction sector 17a connects the main sector 16 to the opening access 11 and a lower junction sector 17b connects the main sector 16 to the main portion 15.

In particular, the main sector 16 is truncated-cone shaped converging towards the main portion 15, and the main sector 16 and the junction sectors 17a,17b are inclined the one to the other.

Advantageously, the main portion 15 is substantially truncated-cone shaped converging by moving away from the reinforced portion 6.

The hollow body 2 comprises a bottom portion 18 associated to the main portion 15.

The main portion 15 extends in the bottom portion 18 in opposite position with respect to the opening access 11.

Usefully, the bottom portion 18 defines the outlet mouth 5.

The bottom portion 18 is substantially dome-shaped at the outlet mouth 5.

The inlet mouth 4 and the outlet mouth 5 allows the connection of the cartridge 1 to the medical equipment.

In particular, the cartridge 1 is connected to an appropriate holder in the medical equipment via the mouths 4,5.

Advantageously, the lid 12 and the bottom portion 18 comprise supporting elements 19 in proximity of the inlet mouth 4 and the outlet mouth 5 respectively.

The supporting elements 19 are of the type of ribs disposed radially around the mouths 4,5, internally the hollow body 2.

Conveniently, the supporting elements 19 increase the stiffness of the cartridge 1 at the mouths 4,5 so as to reduce the risk of breakage during the assembly on the medical equipment.

The supporting elements 19 also have the function of supporting the aforementioned filters at the mouths 4,5.

Moreover, the particular radial arrangement of the supporting elements 19 improves the outflow of the dialysis solution through the outlet mouth 5.

In an embodiment of the present invention, not represented in detail in the figures, the wall of the bottom portion 18 is substantially wave-shaped.

Analogously to what described above for the reinforced portion 6, the surfaces of the bottom portion 18 are both wave-shaped and are provided with a series of indentations and protrusions arranged alternately and in succession the one another.

The thickness of the wave-shaped wall of the bottom portion 18 is substantially constant so as to avoid the local concentration of mechanical stresses and to increase its mechanical resistance.

Moreover, the thickness of the wall of the bottom portion 18 substantially corresponds to the thickness of the wall of the main portion 15.

The operation (not claimed) of the cartridge according to the present invention is as follows.

The buffering agent is inserted in the cavity 3 and the lid 12 is then welded on the flange 13.

The hollow body 2 is, thus, closed in an air-tight manner.

The cartridge 1 is ready to be transported and supplied to a medical staff to be used in a medical equipment for haemodialysis.

The cartridge 1 is then connected to the medical equipment via the inlet mouth 4 and outlet mouth 5.

The particular conformation of the reinforced portion 6 ensures a great resistance of the cartridge 1 to mechanical stresses encountered during transport and connection to the medical equipment.

The medical equipment lets the solvent flow into the cavity 3 through the inlet mouth 4; during the transition through the inlet mouth 4, the solvent is filtered at the same time.

Once in mutual contact, the solvent and the buffering agent form the dialysis solution to be used in the patient's blood treatment.

The dialysis solution flows out from the cavity 3 through the outlet mouth 5 and is filtered at the same time.

It has been ascertained how the described invention achieves the proposed objects and in particular the reinforced portion of the cartridge for the preparation of a dialysis solution highly reduces the risk of breakage and damage due to external stresses.

Indeed, the particular conformation of the reinforced portion avoids the local concentration of mechanical stresses.

Furthermore, the cartridge for the preparation of a dialysis solution has lower manufacturing costs with respect to the cartridges of the known type.

## Claims

1. Cartridge (1) for the preparation of a dialysis solution comprising at least a hollow body (2) defining at least a cavity (3), adapted to contain a buffering agent, and comprising at least an inlet mouth (4) for the inflow of a solvent in said cavity (3) and an outlet mouth (5) for the outflow of said dialysis solution from said cavity (3), in which said hollow body (2) comprises at least a reinforced portion (6) interposed between said inlet mouth (4) and said outlet mouth (5), **characterized in that** said reinforced portion (6) comprises an outer surface (7) and an inner surface (8), said outer surface (7) and said inner surface (8) being both wave-shaped, where the thickness of said wave-shaped wall is substantially constant and said outer surface (7) and said inner surface (8) are substantially smooth and provided with a series of indentations (9) and protrusions (10) arranged alternately and in succession the one another and by the fact that said reinforced portion (6) defines an opening access (11) for introducing said buffering agent into said cavity (3) and that said hollow body (2) comprises a lid (12) associated to said reinforced portion (6) for closing said opening access (11), said lid (12) defining said inlet mouth (4), said lid (12) being separated from the reinforced portion (6).

2. Cartridge (1) according to claim 1, **characterized in that** said hollow body (2) has elongated shape and that said wave-shaped wall develops transversally with respect to the longitudinal axis (A) of said hollow body (2).

3. Cartridge (1) according to one or more of the preceding claims, **characterized in that** said reinforced portion (6) has a substantially circular transversal section, said wave-shaped wall developing along the circumference of said circular transversal section.

4. Cartridge (1) according to one or more of the preceding claims, **characterized in that** said reinforced portion (6) is disposed in proximity of said inlet mouth (4).

5. Cartridge (1) according to one or more of the preceding claims, **characterized in that** said hollow body (2) comprises a main portion (15) associated to said reinforced portion (6), where the thickness of the wall of said main portion (15) substantially corresponds to the thickness of the wall of said reinforced portion (6).

6. Cartridge (1) according to one or more of the preceding claims, **characterized in that** said main portion (15) is substantially truncated-cone shaped and is converging by moving away from said reinforced portion (6).

7. Cartridge (1) according to one or more of the preceding claims, **characterized in that** said reinforced portion (6) comprises a main sector (16) and at least a junction sector (17a, 17b) connecting said main sector (16) to at least one between said opening access (11) and said main portion (15), wherein said junction sector (17a, 17b) is truncated-cone shaped converging towards said main portion (15) and is inclined with respect to said main sector (16).

8. Cartridge (1) according to one or more of the preceding claims, **characterized in that** said reinforced portion (6) comprises two of said junction sectors (17a,17b), of which an upper junction sector (17a) connects said main sector (16) to said opening access (11) and a lower junction sector (17b) connects said main sector (16) to said main portion (15).

9. Cartridge (1) according to one or more of the preceding claims, **characterized in that** said main sector (16) is truncated-cone shaped converging towards said main portion (15), said main sector (16) and said junction sectors (17a, 17b) being inclined the one to the other.

10. Cartridge (1) according to one or more of the preceding claims, **characterized in that** said hollow body (2) comprises a bottom portion (18) associated to said main portion (15), said bottom portion (18) defining said outlet mouth (5).

11. Cartridge (1) according to one or more of the preceding claims, **characterized in that** said bottom portion (18) is substantially dome-shaped at said outlet mouth (5).

12. Cartridge (1) according to one or more of the preceding claims, **characterized in that** the wall of said bottom portion (18) is substantially wave-shaped.

## Patentansprüche

1. Kartusche (1) zur Herstellung einer Dialyselösung, die mindestens einen Hohlkörper (2) umfasst, der mindestens einen Hohlraum (3) definiert, der ausgebildet ist, ein Puffermittel zu enthalten, und der mindestens eine Einlassöffnung (4) für den Zufluss eines Lösungsmittels in den Hohlraum (3) und eine Auslassöffnung (5) für den Abfluss der Dialyselösung aus dem Hohlraum (3) umfasst, wobei der Hohlkörper (2) mindestens einen verstärkten Abschnitt (6) umfasst, der zwischen der Einlassöffnung (4) und der Auslassöffnung (5) angeordnet ist, **dadurch gekennzeichnet, dass** der verstärkte Abschnitt (6) eine Außenfläche (7) und eine Innenfläche (8) umfasst, wobei die Außenfläche (7) und die Innenfläche (8) beide wellenförmig sind, wobei die Dicke der wellenförmigen Wand im Wesentlichen konstant ist und die Außenfläche (7) und die Innenfläche (8) im Wesentlichen glatt und mit einer Reihe von Vertiefungen (9) und Vorsprüngen (10) versehen sind, die abwechselnd und aufeinanderfolgend angeordnet sind, und durch die Tatsache, dass der verstärkte Abschnitt (6) einen Öffnungszugang (11) zum Einführen des Puffermittels in den Hohlraum (3) definiert und dass der Hohlkörper (2) einen Deckel (12) umfasst, der dem verstärkten Abschnitt (6) zugeordnet ist, um den Öffnungszugang (11) zu schließen, wobei der Deckel (12) die Einlassöffnung (4) definiert, wobei der Deckel (12) von dem verstärkten Abschnitt (6) getrennt ist.

2. Kartusche (1) nach Anspruch 1, **dadurch gekennzeichnet, dass** der Hohlkörper (2) eine längliche Form hat und dass sich die wellenförmige Wand quer zu der Längsachse (A) des Hohlkörpers (2) erstreckt.

3. Kartusche (1) nach einem oder mehreren der vorhergehenden Ansprüche, **dadurch gekennzeichnet, dass** der verstärkte Abschnitt (6) einen im Wesentlichen kreisförmigen Querschnitt aufweist, wobei sich die wellenförmige Wand entlang des Umfangs des kreisförmigen Querschnitts erstreckt.

4. Kartusche (1) nach einem oder mehreren der vorhergehenden Ansprüche, **dadurch gekennzeichnet, dass** der verstärkte Abschnitt (6) in der Nähe der Einlassöffnung (4) angeordnet ist.

5. Kartusche (1) nach einem oder mehreren der vorhergehenden Ansprüche, **dadurch gekennzeichnet, dass** der Hohlkörper (2) einen Hauptabschnitt (15) umfasst, der dem verstärkten Abschnitt (6) zugeordnet ist, wobei die Dicke der Wand des Hauptabschnitts (15) im Wesentlichen der Dicke der Wand des verstärkten Abschnitts (6) entspricht.

6. Kartusche (1) nach einem oder mehreren der vorhergehenden Ansprüche, **dadurch gekennzeichnet, dass** der Hauptabschnitt (15) im Wesentlichen kegelstumpfförmig ist und sich von dem verstärkten Abschnitt (6) wegbewegend konvergiert.

7. Kartusche (1) nach einem oder mehreren der vorhergehenden Ansprüche, **dadurch gekennzeichnet, dass** der verstärkte Abschnitt (6) einen Hauptbereich (16) und mindestens einen Verbindungsbereich (17a, 17b) umfasst, der den Hauptbereich (16) mit mindestens einem von dem Öffnungszugang (11) und dem Hauptabschnitt (15) verbindet, wobei der Verbindungsbereich (17a, 17b) kegelstumpfförmig ist und zu dem Hauptabschnitt (15) hin konvergiert und bezüglich des Hauptbereichs (16) geneigt ist.

8. Kartusche (1) nach einem oder mehreren der vorhergehenden Ansprüche, **dadurch gekennzeichnet, dass** der verstärkte Abschnitt (6) zwei der Verbindungsbereiche (17a, 17b) umfasst, von denen ein oberer Verbindungsbereich (17a) den Hauptbereich (16) mit dem Öffnungszugang (11) verbindet und ein unterer Verbindungsbereich (17b) den Hauptbereich (16) mit dem Hauptabschnitt (15) verbindet.

9. Kartusche (1) nach einem oder mehreren der vorhergehenden Ansprüche, **dadurch gekennzeichnet, dass** der Hauptbereich (16) kegelstumpfförmig ist und zum Hauptabschnitt (15) hin konvergiert, wobei der Hauptbereich (16) und die Verbindungsbereiche (17a, 17b) zueinander geneigt sind.

10. Kartusche (1) nach einem oder mehreren der vorhergehenden Ansprüche, **dadurch gekennzeichnet, dass** der Hohlkörper (2) einen Bodenabschnitt (18) umfasst, der dem Hauptabschnitt (15) zugeordnet ist, wobei der Bodenabschnitt (18) die Auslassöffnung (5) definiert.

11. Kartusche (1) nach einem oder mehreren der vorhergehenden Ansprüche, **dadurch gekennzeichnet, dass** der Bodenabschnitt (18) an der Auslassöffnung (5) im Wesentlichen kuppelförmig ist.

12. Kartusche (1) nach einem oder mehreren der vorhergehenden Ansprüche, **dadurch gekennzeichnet, dass** die Wand des Bodenabschnitts (18) im Wesentlichen wellenförmig ist.

## Revendications

1. - Cartouche (1) pour la préparation d'une solution de dialyse comprenant au moins un corps creux (2) définissant au moins une cavité (3), agencée pour contenir un agent tampon, et comprenant au moins une bouche d'entrée (4) pour l'entrée d'un solvant dans ladite cavité (3) et une bouche de sortie (5) pour la sortie de ladite solution de dialyse à partir de ladite cavité (3), ledit corps creux (2) comprenant au moins une partie renforcée (6) interposée entre ladite bouche d'entrée (4) et ladite bouche de sortie (5), **caractérisée par le fait que** ladite partie renforcée (6) comprend une surface extérieure (7) et une surface intérieure (8), ladite surface extérieure (7) et ladite surface intérieure (8) étant toutes deux en forme de vague, l'épaisseur de ladite paroi en forme de vague étant sensiblement constante, et ladite surface extérieure (7) et ladite surface intérieure (8) sont sensiblement lisses et pourvues d'une série d'indentations (9) et de saillies (10) disposées alternativement et successivement les unes aux autres, et **par le fait que** ladite partie renforcée (6) définit un accès d'ouverture (11) pour introduire ledit agent tampon dans ladite cavité (3) et que ledit corps creux (2) comprend un couvercle (12) associé à ladite partie renforcée (6) pour fermer ledit accès d'ouverture (11), ledit couvercle (12) définissant ladite bouche d'entrée (4), ledit couvercle (12) étant séparé de la partie renforcée (6).

2. - Cartouche (1) selon la revendication 1, **caractérisée par le fait que** ledit corps creux (2) a une forme allongée et que ladite paroi en forme de vague se développe transversalement par rapport à l'axe longitudinal (A) dudit corps creux (2).

3. - Cartouche (1) selon une ou plusieurs des revendications précédentes, **caractérisée par le fait que** ladite partie renforcée (6) a une section transversale sensiblement circulaire, ladite paroi en forme de vague se développant sur la circonférence de ladite section transversale circulaire.

4. - Cartouche (1) selon une ou plusieurs des revendications précédentes, **caractérisée par le fait que** ladite partie renforcée (6) est disposée à proximité de ladite bouche d'entrée (4).

5. - Cartouche (1) selon une ou plusieurs des revendications précédentes, **caractérisée par le fait que** ledit corps creux (2) comprend une partie principale (15) associée à ladite partie renforcée (6), l'épaisseur de la paroi de ladite partie principale (15) correspondant sensiblement à l'épaisseur de la paroi de ladite partie renforcée (6).

6. - Cartouche (1) selon une ou plusieurs des revendications précédentes, **caractérisée par le fait que** ladite partie principale (15) est sensiblement tronconique et converge en s'éloignant de ladite partie renforcée (6).

7. - Cartouche (1) selon une ou plusieurs des revendications précédentes, **caractérisée par le fait que** ladite partie renforcée (6) comprend un secteur principal (16) et au moins un secteur de jonction (17a, 17b) reliant ledit secteur principal (16) à au moins l'un parmi ledit accès d'ouverture (11) et ladite partie principale (15), ledit secteur de jonction (17a, 17b) étant tronconique, convergeant vers ladite partie principale (15) et étant incliné par rapport audit secteur principal (16).

8. - Cartouche (1) selon une ou plusieurs des revendications précédentes, **caractérisée par le fait que** ladite partie renforcée (6) comprend deux desdits secteurs de jonction (17a, 17b), dont un secteur de jonction supérieur (17a) qui relie ledit secteur principal (16) audit accès d'ouverture (11) et un secteur de jonction inférieur (17b) qui relie ledit secteur principal (16) à ladite partie principale (15).

9. - Cartouche (1) selon une ou plusieurs des revendications précédentes, **caractérisée par le fait que** ledit secteur principal (16) est tronconique, convergeant vers ladite partie principale (15), ledit secteur principal (16) et lesdits secteurs de jonction (17a, 17b) étant inclinés l'un par rapport à l'autre.

10. - Cartouche (1) selon une ou plusieurs des revendications précédentes, **caractérisée par le fait que** ledit corps creux (2) comprend une partie inférieure (18) associée à ladite partie principale (15), ladite partie inférieure (18) définissant ladite bouche de sortie (5).

11. - Cartouche (1) selon une ou plusieurs des revendications précédentes, **caractérisée par le fait que** ladite partie inférieure (18) est sensiblement en forme de dôme à ladite bouche de sortie (5).

12. - Cartouche (1) selon une ou plusieurs des revendications précédentes, **caractérisée par le fait que** la paroi de ladite partie inférieure (18) est sensiblement en forme de vague.
